# EUROPEAN PATENT SPECIFICATION

(11) **EP 2 501 363 B1**
(45) Date of publication and mention of the grant of the patent: **14.01.2015**
(21) Application number: 10795437.2
(22) Date of filing: 17.11.2010
(51) Int. Cl.: A61K 9/12, A61K 31/46, A61P 11/06, A61K 31/439

(54) **INHALATION SOLUTIONS**
INHALATIONSLÖSUNGEN
SOLUTIONS D'INHALATION

(30) Priority: 17.11.2009 IN MU26572009
(43) Date of publication of application: 26.09.2012
(73) Proprietor: Cipla Limited, Mumbai - 400013 (IN)
(72) Inventor: LULLA, Amar, Mumbai 400 005 Maharashtr (IN); MALHOTRA, Geena, Mumbai 400 010 Maharashtra (IN)
(74) Representative: Turner, Craig Robert
(86) International application number: PCT/GB2010/002126
(87) International publication number: WO 2011/061498

(56) References cited:
- WO-A1-2004/019985
- WO-A2-01/62227
- WO-A2-2006/040598
- US-A1- 2005 058 606
- BELL JOHN ET AL: "The rejuvenated pressurised metered dose inhaler", EXPERT OPINION ON DRUG DELIVERY, INFORMA HEALTHCARE, GB, vol. 4, no. 3, 1 May 2007 (2007-05-01), pages 215-234, XP009150530, ISSN: 1742-5247

## Description

### Technical field of Invention

The present invention relates to pressurized inhalation solutions and the process of preparing the same and their use for the treatment of asthma, COPD (chronic obstructive pulmonary disease) and other respiratory disorders.

### Background & Prior art

The knowledge of size and distribution of particles produced from aerosol formulations is important not only from the viewpoint of product optimization but also from potential inhalation characteristics such as certain actuator designs, valve characteristics, canister properties, etc.

The administration of aerosol formulations of medicaments by means of pressurized, metered-dose inhalers (MDIs) is used widely in therapy, such as in the treatment of obstructive airway diseases and asthma. Compared with oral administration, inhalation provides more rapid onset of action while minimizing systemic side effects. Aerosol formulations can be administered by inhalation through the mouth or topically by application to the nasal mucosa.

Formulations for aerosol administration via MDIs can be solutions or suspensions. Solution formulations offer the advantage of being homogeneous in nature with the medicament and excipient completely dissolved in the propellant vehicle. Solution formulations also obviate physical stability problems associated with suspension formulations and thus assure more consistent uniform dosage administration while also eliminating the need for surfactants.

The administration of aerosol solution formulations via MDIs is dependent upon the propulsive force of the propellant system used in its manufacture. Traditionally, the propellant comprised a mixture of chlorofluorocarbons (CFCs) to provide the desired solubility, vapor pressure, and stability of the formulation. However, since it has been established in recent years that CFCs are environmentally harmful because they contribute to the depletion of the Earth's ozone layer, it is desirable to substitute environmentally safe hydrofluorocarbon (HFC) propellants or other non-chlorinated propellants for environmentally harmful CFC propellants in aerosol inhalation formulations. For example, U.S. Patent No. 4,174,295 discloses the use of propellant systems consisting of combinations of HFCs, which may also contain a saturated hydrocarbon component, suitable for application in the fields of home products such as hair lacquers, anti-perspiration products, perfumes, deodorants, paints, insecticides and the like.

Many of these applications, in which HFAs are used as propellant, propose the addition of one or more of adjuvants including compounds acting as co-solvents, surface active agents including fluorinated and non-fluorinated surfactants, dispersing agents including alkylpolyethoxylates and stabilizers.

The effectiveness of an aerosol device, for example an MDI, is a function of the dose deposited at the appropriate site in the lungs. Deposition is affected by several parameters, of which the most important are the Fine Particle Dose (FPD) and the aerodynamic particle size. Solid particles and/or droplets in an aerosol formulation can be characterized by their mass median aerodynamic diameter (MMAD, the diameter around which the mass aerodynamic diameters are distributed equally). The FPD gives a direct measure of the mass of particles within a specified size range and is closely related to the efficacy of the product.

In the international application no PCT/EP98/03533 filed on Oct. 6, 1998 the applicant discloses solution compositions for use in an aerosol inhaler, comprising an active material, a propellant containing a hydrofluoroalkane (HFA), a cosolvent and further comprising a low volatility component to increase the mass median aerodynamic diameter (MMAD) of the aerosol particles on actuation of the inhaler.

WO 98/34596 discloses solution compositions for use in an aerosol inhaler, comprising an active material, a propellant containing a hydrofluoroalkane (HFA), a cosolvent and further comprising a low volatility component to increase the mass median aerodynamic diameter (MMAD) of the aerosol particles on actuation of the inhaler. Said application does not address the technical problem of the chemical stability of the active ingredient but it rather concerns the drug delivery to lungs.

The widespread use of these formulations is limited by their chemical instability, causing the formation of degradation products.

U.S. Patent No. 5, 676, 930 proposes the use of acids as stabilizers preventing the chemical degradation of the active ingredient in aerosol solution formulations comprising HFAs. Among the selected medicaments ipratropium bromide is disclosed, for which many composition examples are supplied, in which the active ingredient is in combination with an organic or inorganic acid.

WO96/32099, WO96/32150, WO96/32151 and WO96/32345 disclose metered dose inhalers for the administration of different active ingredients in suspension in the propellant, wherein the internal surfaces of the inhaler are partially or completely coated with one or more fluorocarbon polymers optionally in combination with one or more non-fluorocarbon polymers.

It is also widely known that the aerosol formulations of the above mentioned kind are often influenced by reducing the particle size, by reducing the drug concentration, by including additives like surfactants in the formulation, by increasing vapor pressure and thereby play a major role in identifying the therapeutic efficacy which is to be achieved in patients as discussed in Journal of Pharmaceutical Sciences Vol. 58, No. 4, April 1969 titled "Influence of formulation on Aerosol Particle Size" by Polli et. al*.*

Anticholinergic quaternary ammonium salts, such as oxitropium bromide, tiotropium bromide and ipratropium bromide, are usually prescribed in the form of inhalatory formulations, for patients suffering from respiratory disorders, due to their bronchodilating, antisecretive and bronchospasm-preventive actions.

Said drugs, particularly ipratropium bromide, induce less prompt bronchodilation than conventional .beta.2-agonists, but provide greater peak response and longer duration of action. Said characteristics make them particularly suitable for the chronic treatment rather than the acute one (Ferguson G. et al. N Engl J Med 1993, 328, 1017-1022).

Although the single optimal dose for the administration of nebulized ipratropium bromide in the treatment of COPD has been established to be 0.4 mg (Gross N J et al Am Rev Respir Dis 1989, 139, 1188-1191), the dosage via pressurized metered dose inhalers has not yet been unquivocally established. Some authors (Ferguson G. et al, passim) have however suggested that treatment of said disease could benefit from use of higher doses than recommended ones (54-109 micrograms). Recent studies (Ikeda A et al. Thorax 1996, 51, 48-53; Shivaram U et al. Resp Med 1997, 91, 327-334; Wood F et al. Amer J Resp Crit Care Med 1999, 159, A 523) have demonstrated that the administration of single doses ranging from 80 to 320 micrograms is beneficial for the improvement in lung function, maximal workload and oxygen consumption.

However, none of the above art teaches reducing the dose of the active to be administered through metered dose inhalers without compromising on the FPD of the active particles/ aerosol particles in the requisite formulation stressing particularly on the parameters as discussed in the international application no PCT/EP98/03533. Particularly, it was surprisingly found that administration of pressurized aerosol inhalable formulation in the form of solution comprising an active through low orifice actuator resulted in the desired FPD of the active particles/ aerosol particles.

Hence, there exists a need to develop a pressurized aerosol inhalable formulation achieving desirable FPD of the active particles/aerosol particles in the said formulation.

### Object of the Invention

An object of the present invention is to provide an inhalable aerosol formulation comprising an effective amount of an active(s) or physiologically acceptable salt thereof along with pharmaceutically acceptable excipients.

Another object is to provide a process for the preparation of an inhalable formulation comprising an effective amount of an active(s) or physiologically acceptable salt thereof along with pharmaceutically acceptable excipients.

Yet another object of the present invention is to provide a method for prophylaxis or treatment of asthma, COPD or related respiratory disorders which comprises administering an effective amount of an active(s) or physiologically acceptable salt thereof along with 5 pharmaceutically acceptable excipients.

### Summary of the Invention

According to a first aspect of the present invention there is provided an inhaler for an inhalation formulation, comprising a canister containing a pharmaceutical composition under pressure; a metering valve for measuring a metered dose of the composition from the canister for administration to a patient in need thereof; and an actuator for actuating discharge of the metered dose to the patient; wherein the actuator includes a discharge orifice having a diameter in the range 0.2 to 0.4 mm; and wherein the pharmaceutical composition comprises an anticholinergic agent and a pharmaceutically acceptable propellant, characterized in that the metering valve is configured to dispense the metered dose of the pharmaceutical composition containing 2.5 to 18 micrograms of the or each anticholinergic agent.

Also disclosed in the present invention is an inhalation solution comprising:
i) an anticholinergic selected from the group consisting of tiotropium, ipratropium, oxitropium, aclidinium or their pharmaceutically acceptable salts, solvates, tautomers, derivatives, enantiomers, isomers, hydrates, prodrugs or polymorphs thereof
ii) one or more pharmaceutically acceptable excipients.

Also disclosed in the present invention is an inhalation solution comprising tiotropium or its pharmaceutically acceptable salts, solvates, tautomers, derivatives, enantiomers, isomers, hydrates, prodrugs or polymorphs thereof with one or more pharmaceutically acceptable excipients comprising an HFC propellant and a co-solvent.

Also disclosed in the present invention is a method of administering an inhalation solution comprising tiotropium or its pharmaceutically acceptable salts, solvates, tautomers, derivatives, enantiomers, isomers, hydrates, prodrugs or polymorphs thereof with one or more pharmaceutically acceptable excipients comprising an HFC propellant and a co-solvent administered through metered dose inhaler.

Also disclosed in the present an inhalation solution comprising tiotropium or its pharmaceutically acceptable salts, solvates, tautomers, derivatives, enantiomers, isomers, hydrates, prodrugs or polymorphs thereof and another active ingredient comprising beta adrenergic agonists and/or corticosteroids with one or more pharmaceutically acceptable excipients comprising an HFC propellant and a co-solvent administered through metered dose inhaler.

### Detailed description of the Invention

As discussed herein, the inventors have surprisingly found that administration of pressurized aerosol inhalable formulation in the form of solution comprising an active through low 20 orifice actuator resulted in the desired FPD of the active particles/ aerosol particles.

The low orifice actuator advantageously has an orifice diameter ranging from 0.2 mm to 0.4 mm. Preferably the diameter is from 0.2 mm to 0.33 mm, more preferably 0.28 mm to 0.33 mm.

The inhalable solution, according to the present invention, may comprise one or more anticholinergic agent comprising tiotropium, ipratropium, oxitropium, aclidinium or their pharmaceutically acceptable salts, solvates, tautomers, derivatives, enantiomers, isomers, hydrates, prodrugs or polymorphs thereof

A preferred salt is the bromide salt especially the bromide salts of tiotropium, ipratropium, oxitropium and aclidinium. In a particularly preferred embodiment, according to the present invention, the inhalation solution may comprise tiotropium or a salt thereof, most preferably tiotropium bromide, as the active with one or more pharmaceutically acceptable excipients.

According to the present invention, suitable pharmaceutically acceptable excipients may comprise one or more HFC propellants, co-solvents, low volatility component, stabilizers, dispersing agents, pH adjusting agents, antioxidants, preservatives, chelating agents, surface active agents or mixtures thereof.

A small amount of water (up to about 1% by weight) may also be present in the propellant/cosolvent system.

Suitable HFC propellants are those which, when mixed with the cosolvent(s), form a homogeneous propellant system in which a therapeutically effective amount of the medicament can be dissolved. The HFC propellant must be toxicologically safe and must have a vapor pressure which is suitable to enable the medicament to be administered via a pressurized MDI. Additionally, the HFC propellant must be compatible with the components of the MDI device (such as containers, valves, and sealing gaskets, etc.) which is employed to administer the medicament. Preferred HFC propellants are 1,1,1,2-tetrafluoroethane (HFC-134(a)) and 1,1,1,2,3,3,3,-heptafluoropropane (HFC-227). HFC-134(a) is particularly preferred. Other examples of HFC propellants are HFC-32 (difluoromethane), HFC-143(a) (1,1,1-trifluoroethane), HFC-134 (1,1,2,2-tetrafluoroethane), and HFC-152a (1,1-difluoroethane).

It will be apparent to those skilled in the art that non-halogenated hydrocarbon propellants may be used in place of the HFC propellants in the present invention. Examples of non-halogenated hydrocarbons are saturated hydrocarbons, including propane, n-butane, and isobutane, and ethers, including diethyl ether.

It will also be apparent to those skilled in the art that, although the use of a single HFC propellant is preferred, a mixture of two or more HFC propellants, or a mixture of at least one HFC propellant and one or more non-CFC propellants, may be employed in the aerosol solution formulation of the present invention.

Suitable cosolvents that may be employed in the inhalation solution may comprise one or more polar cosolvent such as C₂₋₆ aliphatic alcohols and polyols, for example ethanol, isopropanol, propylene glycol. The co-solvent is preferably present in an amount from 15-20 wt% of the formulation.

Suitably, the low volatility component that may be employed in the inhalation solution may comprises a polyol preferably, glycerol, isopropyl myristate. The low volatility component may be present in a range of 0.5-1% of the formulation.

Suitably the inhalation solution may comprise other substances, for example, polyoxyethylene alcohols, and polyoxyethylene fatty acid esters;
Suitably the preservatives that may be employed in the inhalation solution may be present in a range of 0.01-0.03% of the formulation. The preservative that may be employed in the inhalation solution may comprise benzalkonium chloride
Suitably the chelating agents that may be employed in the inhalation solution may be present in a range of 0.0002-0.001% of the formulation. The chelating agents that may be employed in the inhalation solution may comprise EDTA
Suitably, the pH adjusting agent that may be employed in the inhalation solution may comprise organic or inorganic acids e.g. hydrochloric acid, citric acid, etc.

One or more surfactants may be employed which may serve to stabilize the inhalation solutions and provide lubrication to the valve system of the metered dose inhaler. Some of the most commonly employed surfactants may comprise oils (e.g. corn oil, olive oil, etc), phospholipids such as lecithins, acids such as oleic acid.

The inhalation solutions, according to the present invention, may further comprise one or more active agents selected from beta adrenergic agonists or corticosteroids or their pharmaceutically acceptable salts, solvates, tautomers, derivatives; enantiomers, isomers, hydrates, prodrugs or polymorphs thereof.

According to a preferred embodiment of the present invention, the inhalable solution may comprise an anticholinergic agent or its salts, solvates, tautomers, derivatives, enantiomers, isomers, hydrates, prodrugs or polymorphs thereof, preferably, tiotropium, ipratropium, oxitropium, aclidinium or their pharmaceutically acceptable salts, solvates, tautomers, derivatives, enantiomers, isomers, hydrates, prodrugs or polymorphs thereof, more preferably tiotropium bromide to be used with a pressurized metered dose inhaler, comprising a metering valve and low orifice actuator ranging from 0.2 mm to 0.4 mm diameter (preferably 0.2 to 0.33 mm, more preferably 0.28 to 0.33 mm) characterized by a desirable FPD of the said active particles/ aerosol particles.

According to a preferred embodiment of the present invention, the inhalable solution may comprise tiotropium or their pharmaceutically acceptable salts, solvates, tautomers, derivatives, enantiomers, isomers, hydrates, prodrugs or polymorphs thereof, more preferably tiotropium bromide to be used with a pressurized metered dose inhaler, wherein the said tiotropium concentration corresponds to single doses ranging from 2.5 micrograms to 18 micrograms, preferably 2.5 to 15 micrograms, more preferably 4.5 to 9 micrograms, characterized by a desirable FPD of the said active particles/ aerosol particles

Preferably, the inhalable solution may comprise tiotropium bromide having a single dose ranging from 2.5 micrograms to 18 micrograms, preferably 2.5 to 15 micrograms, more preferably 4.5 to 9 micrograms, with one or more pharmaceutically acceptable excipients comprising one or more co-solvents, low volatility component, HFC propellants or mixtures thereof to be used with metered dose inhaler comprising a metering valve and low orifice actuator ranging from 0.2 mm to 0.4 mm diameter (preferably 0.2 to 0.33 mm, more preferably 0.28 to 0.33 mm) characterized by a desirable FPD of the said active particles/ aerosol particles.

Preferably, the inhalable solution may comprise an anticholinergic agent or its salts, solvates, tautomers, derivatives, enantiomers, isomers, hydrates, prodrugs or polymorphs thereof, preferably, tiotropium, ipratropium; oxitropium, aclidinium or their pharmaceutically acceptable salts, solvates, tautomers, derivatives, enantiomers, isomers, hydrates, prodrugs or polymorphs thereof, more preferably tiotropium bromide and one or more of beta adrenergic agent(s) or corticosteroids or both to be used with a pressurized metered dose inhaler comprising a metering valve and low orifice actuator ranging from 0.2 mm to 0.4 mm diameter (preferably 0.2 to 0.33 mm, more preferably 0.28 to 0.33 mm) characterized by a desirable FPD of the said active particles/ aerosol particles.

According to a preferred embodiment of the present invention, the inhalable solution may comprise tiotropium or their pharmaceutically acceptable salts, solvates, tautomers, derivatives, enantiomers, isomers, hydrates, prodrugs or polymorphs thereof, more preferably tiotropium bromide and one or more of beta adrenergic agent(s) or corticosteroids or both to be used with a pressurized metered dose inhaler to be used with a pressurized metered dose inhaler, wherein the said tiotropium concentration corresponds to single doses ranging from 2.5 micrograms to 18 micrograms, preferably 2.5 to 15 micrograms, more preferably 4.5 to 9 micrograms, characterized by a desirable FPD of the said active particles/ aerosol particles.

Preferably, the inhalable solution may comprise tiotropium bromide having a single dose ranging from 2.5 micrograms to 18 micrograms, preferably 2.5 to 15 micrograms, more preferably 4.5 to 9 micrograms, with one or more pharmaceutically acceptable excipients and one or more of beta adrenergic agent(s) or corticosteroids or both with one or more co-solvents, low volatility component, HFC propellants or mixtures thereof to be used with metered dose inhaler comprising a metering valve and low orifice actuator ranging from 0.2 mm to 0.4 mm diameter (preferably 0.2 to 0.33 mm, more preferably 0.28 to 0.33 mm) characterized by a desirable FPD of the said active particles/ aerosol particles.

According to another embodiment of the present invention, there is provided a method of administering an anticholinergic agentor its salts, solvates, tautomers, derivatives, enantiomers, isomers, hydrates, prodrugs or polymorphs thereof preferably, tiotropium, ipratropium, oxitropium, aclidinium or their pharmaceutically acceptable salts, solvates, tautomers, derivatives, enantiomers, isomers, hydrates, prodrugs or polymorphs thereof, more preferably tiotropium bromide with one or more pharmaceutically acceptable excipients comprising HFC propellants, co-solvents, low volatility component, stabilizers, dispersing agents, pH adjusting agents, surface active agents or mixtures thereof, to be used with metered dose inhaler comprising a metering valve and low orifice actuator ranging from 0.2 mm to 0.4 mm diameter (preferably 0.2 to 0.33 mm, more preferably 0.28 to 0.33 mm) characterized by a desirable FPD of the said active particles/ aerosol particles.

According to another embodiment of the present invention, there is provided a method of administering tiotropium or its pharmaceutically acceptable salts, solvates, tautomers, derivatives, enantiomers, isomers, hydrates, prodrugs or polymorphs thereof, more preferably tiotropium bromide and one or more of beta adrenergic agent(s) or corticosteroids or both to be used with a pressurized metered dose inhaler, wherein the said tiotropium concentration corresponds to single doses ranging from 2.5 micrograms to 18 micrograms, preferably 2.5 to 15 micrograms, more preferably 4.5 to 9 micrograms, characterized by a desirable FPD of the said active particles/ aerosol particles.

Preferably, there is provided a method of administering tiotropium bromide having a single dose ranging from 2.5 micrograms to 18 micrograms, preferably 2.5 to 15 micrograms, more preferably 4.5 to 9 micrograms, with one or more pharmaceutically acceptable excipients comprising one or more co-solvents, low volatility component, HFC propellants or mixtures thereof to be used with metered dose inhaler comprising a metering valve and low orifice actuator ranging from 0.2 mm to 0.4 mm diameter (preferably 0.2 to 0.33 mm, more preferably 0.28 to 0.33 mm) characterized by a desirable FPD of the said active particles/ aerosol particles.

### Brief Description of the Drawing

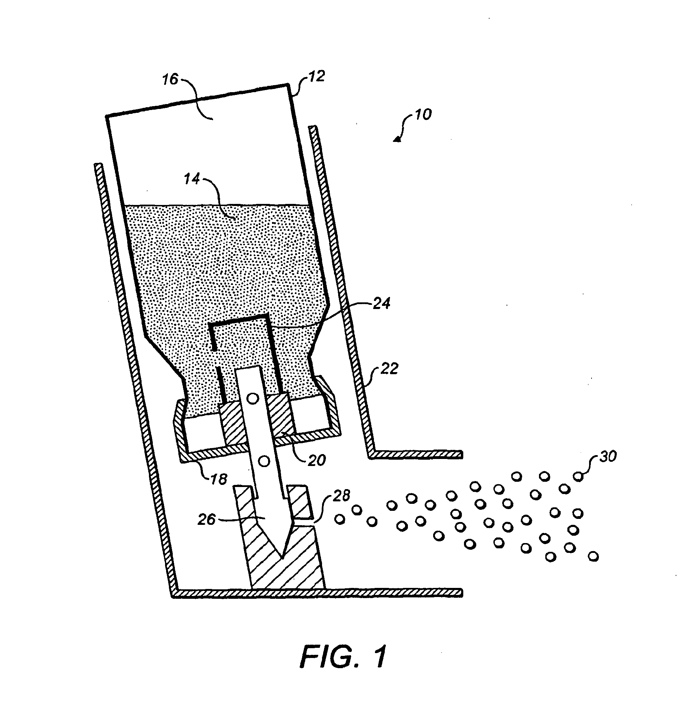

Reference is made to Fig. 1 which is a cross sectional view of an embodiment of an inhaler according to the invention.

### Detailed Description of the Drawing

Referring to Fig. 1 a typical embodiment of the invention is disclosed in the form of a metered-dose inhaler (MDI) generally designated 10.

The inhaler 10 comprises a canister 12, which contains a liquid pharmaceutical composition 14 comprising an anticholinergic agent. A retaining cup 24 is also present within the canister, which engages a metering valve 18. A pressurised gas phase 16 is present within the canister 12, the pressure of which drives the composition 14 from the canister 12 in metered doses, when the metering valve 18 is opened by an actuator 22.

The metering valve 18 has a metering chamber 20 which contains the required dose of the composition 14. The metering valve 18 serves to deliver the required dose of the composition 14, when actuated by the actuator 22. As noted above, the required dose of the anticholinergic agent in the composition 14 is preferably 2.5 to 18 micrograms.

The actuator 22 is operated by a patient to deliver the required dose. The dose flows from the metering chamber 20 to an expansion chamber 26 of the actuator 22, and through an actuator nozzle 28. The expansion of the composition 14 in the expansion chamber 26, followed by flow through the actuator nozzle 28, forms a high velocity spray 30 of the composition 14 for delivery to the patient.

The structure of the inhaler as shown in Fig.1 is conventional, and its operation would be well understood by a person skilled in the art. However, we have found that by providing the actuator nozzle 28 with a diameter in the range 0.2 to 0.4 mm, the required dosage of the anticholinergic agent can be lower than expected (in particular, 2.5 to 18 micrograms, preferably 2.5 to 15 micrograms, more preferably 4.5 to 9 micrograms,), yet still provide an effective FPD.

### Examples

The following examples are for the purpose of illustration of the invention only and are not intended in any way to limit the scope of the present invention.

### Example I

| **Sr. No.** | **Ingredients** | **Quantity/ Can** |
|---|---|---|
| 1 | Tiotropium Bromide Monohydrate | 2.5/ 4.5/ 9/18 micrograms per spray |
| 2 | Benzalkonium Chloride -50% solution | 0.02% of the formulation |
| 3 | Disodium EDTA | 0.001% of the formulation |
| 4 | Purified water | 0.5% of the formulation |
| 5 | Glycerol | 1% of the formulation |
| 6 | Ethanol | 15% of the formulation |
| 7 | Hydrochloric acid 1N | q. s. to adjust pH between 2.7 to 3.1 |
| 8 | HFA 134a | q. s. |

### Manufacturing process:

1) Disodium EDTA was dissolved in purified water
2) The above solution is added to ethanol containing Benzalkonium chloride & glycerol and pH was adjusted between 2.7 to 3.1 with the help of 1N HCL
3) Tiotropium bromide monohydrate was added to the above solution & mixed to dissolve followed by can filling and crimping with a suitable metering valve.
4) Charge HFA -134a propellant was charged through the valve.

### Example II

| **Sr. No.** | **Ingredients** | **Quantity/ Can** |
|---|---|---|
| 1 | Tiotropium Bromide Monohydrate | 2.5/ 4.5/ 9/ 18 micrograms per spray |
| 2 | Purified water | 0.5% of the formulation |
| 3 | Glycerol | 1% of the formulation |
| 4 | Ethanol | 15% of the formulation |
| 5 | Citric acid anhydrous | q. s. to adjust pH between 2.7 to 3.1 |
| 6 | HFA 134a | q. s. |

### Manufacturing process:

1) Glycerol was dissolved in purified water and ethanol.
2) pH was adjusted between 2.7 to 3.1 with the help of citric acid anhydrous.
3) Tiotropium bromide monohydrate was added to the above solution & mixed to dissolve followed by can filling and crimping with a suitable metering valve.
4) Charge HFA -134a propellant was charged through the valve.

### Example III

| **Sr. No.** | **Ingredients** | **Quantity/ Can** |
|---|---|---|
| 1 | Tiotropium Bromide Monohydrate | 2.5/ 4.5/ 9/ 18 micrograms per spray |
| 2 | Purified water | 0.5% of the formulation |
| 3 | Ethanol | 20% of the formulation |
| 4 | Citric acid anhydrous | q. s. to adjust pH between 2.7 to 3.1 |
| 5 | HFA 134a | q. s. |

### Manufacturing process:

1) Purified water was dissolved in ethanol.
2) pH was adjusted between 2.7 to 3.1 with the help of citric acid anhydrous.
3) Tiotropium bromide monohydrate was added to the above solution & mixed to dissolve followed by can filling and crimping with a suitable metering valve.
4) Charge HFA -134a propellant was charged through the valve.

### Example IV

| **Sr. No.** | **Ingredients** | **Quantity/ Can** |
|---|---|---|
| 1 | Tiotropium Bromide Monohydrate | 2.5/ 4.5/ 9/ 18 micrograms per spray |
| 2 | Ethanol | 20% of the formulation |
| 3 | Citric acid anhydrous | q. s. to adjust pH between 2.7 to 3.1 |
| 4 | HFA 134a | q. s. |

### Manufacturing process:

1) Citric acid was dissolved in ethanol to adjust the pH between 2.7 to 3.1.
2) Tiotropium bromide monohydrate was added to the above solution & mixed to dissolve followed by can filling and crimping with a suitable metering valve.
4) Charge HFA -134a propellant was charged through the valve.

### Example V

| **Sr. No.** | **Ingredients** | **Quantity/ Can** |
|---|---|---|
| 1 | Tiotropium Bromide Monohydrate | 2.5/ 4.5/ 9/18 micrograms per spray |
| 2 | Purified water | 0.5% of the formulation |
| 3 | Ethanol | 15% of the formulation |
| 4 | Citric acid anhydrous | q. s. to adjust pH between 2.7 to 3.1 |
| 5 | HFA 134a | q. s. |

### Manufacturing process:

1) Purified water was dissolved in ethanol.
2) pH was adjusted between 2.7 to 3.1 with the help of citric acid anhydrous.
3) Tiotropium bromide monohydrate was added to the above solution & mixed to dissolve followed by can filling and crimping with a suitable metering valve.
4) Charge HFA -134a propellant was charged through the valve.

### Example VI

| **Sr. No.** | **Ingredients** | **Quantity/ Can** |
|---|---|---|
| 1 | Tiotropium Bromide Monohydrate | 2.5/ 4.5/ 9/ 18 micrograms per spray |
| 2 | Ethanol | 15% of the formulation |
| 3 | Citric acid anhydrous | q. s. to adjust pH between 2.7 to 3.1 |
| 4 | HFA 134a | q. s. |

### Manufacturing process:

1) Citric acid was dissolved in ethanol to adjust the pH between 2.7 to 3.1.
2) Tiotropium bromide monohydrate was added to the above solution & mixed to dissolve followed by can filling and crimping with a suitable metering valve.
3) Charge HFA -134a propellant was charged through the valve.

### Example VII

A series of tests were carried out to demonstrate the effectiveness of the inhaler with an orifice in the range 0.2 to 0.4 mm.

### Test 1

| | |
|---|---|
| **PRODUCT NAME:** | **TIOTROPIUM BR HFA INHALER (4.5 MCG/SP) 120MD** |
| **CAN TYPE:** | **19ml ANODISED** |
| **VALVE TYPE:** | **50mcl PE** |
| **Formulation:** | **15% Ethanol + 0.5% Water + Citric acid + HFA134a** |

The results of the test are shown in Table 1. The fine particle mass (FPM) results were determined by cascade impactor and the results are given in micrograms.

### Test 2

| | |
|---|---|
| **PRODUCT NAME:** | **TIOTROPIUM BR HFA INHALER (4.5 MCG/SP) 120MD** |
| **CAN TYPE:** | **19ml ANODISED** |
| **VALVE TYPE:** | **50mcl PE** |
| **Formulation:** | **15% Ethanol + Citric acid + HFA134a** |

The results of the test are shown in Table 2. The fine particle mass (FPM) results were determined by cascade impactor and the results are given in micrograms.

### Test 3

| | |
|---|---|
| **PRODUCT NAME:** | **TIOTROPIUM BR HFA INHALER (4.5 MCG**/**SP**) **120MD** |
| **CAN TYPE:** | **19ml ANODISED** |
| **VALVE TYPE:** | **50mcl PE** |
| **Formulation:** | **15% Ethanol+0.5% water+1%glycesol+Citric acid + HFA 134a** |

The results of the test are shown in Table 3. The fine particle mass (FPM) results were determined by cascade impactor and the results are given in micrograms.

### Test 4

| | |
|---|---|
| **PRODUCT NAME:** | **TIOTROPIUM BR HFA INHALER (4.5 MCG/SP) 120MD** |
| **CAN TYPE:** | **19ml ANODISED** |
| **VALVE TYPE:** | **50mcl PE** |
| **Formulation:** | **15% Ethanol+0.5% water + BKC + Disodium EDTA+ 1N HCL + HFA134a** |

The results of the test are shown in Table 4. The fine particle mass (FPM) results were determined by cascade impactor and the results are given in micrograms.

**Table 1**

| **ACTUATOR** | **0.28 mm** | | | **0.30 mm** | | |
|---|---|---|---|---|---|---|
| | CAN-1 | CAN-2 | CAN-3 | CAN-1 | CAN-2 | CAN-3 |
| **MB*** **(mcg)** | 4.01 | 4.01 | 3.88 | 4.13 | 4.17 | 4.11 |
| **FPD** | 1.44 | 1.42 | 1.42 | 1.11 | 1.14 | 1.16 |
| **MMAD(mcg)** | 1.3 | 1.20 | 1.20 | 1.4 | 1.3 | 1.4 |

| | | | | | | |
|---|---|---|---|---|---|---|
| * Mass Balance | | | | | | |

**Table 2**

| **ACTUATOR** | **0.28 mm** | | | **0.33 mm** | | | **0.48 mm** | | **0.58 mm** | |
|---|---|---|---|---|---|---|---|---|---|---|
| | CAN-1 | CAN-2 | CAN-3 | CAN-1 | CAN-2 | CAN-3 | CAN-1 | CAN-2 | CAN-1 | CAN-2 |
| **MB (mcg)** | 3.67 | 3.86 | 3.73 | 3.92 | 4.23 | 4.15 | 4.18 | 4.37 | 4.43 | 4.26 |
| **FPD** | 1.31 | 1.27 | 1.29 | 1.05 | 1.05 | 1.05 | 0.57 | 0.65 | 0.48 | 0.5 |
| **MMAD(mcg)** | 1.1 | 1.2 | 1.10 | 1.3 | 1.3 | 1.3 | 1.4 | 1.3 | 1.6 | 1.4 |

**Table 3**

| **ACTUATOR** | **0.28 mm** | | |
|---|---|---|---|
| | CAN-1 | CAN-2 | CAN-3 |
| **MS** (**mcg**) | 3.79 | 3.97 | 3.88 |
| **FPD** | 1.00 | 0.99 | 1.02 |
| **MMAD(mcg)** | 2.7 | 2.7 | 2.7 |

**Table 4**

| **ACTUATOR** | **0.28 mm** | | |
|---|---|---|---|
| | CAN-1 | CAN-2 | CAN-3 |
| **MB (mcg)** | 3.85 | 3.80 | 3.76 |
| **FPD** | 1.18 | 1.19 | 1.12 |
| **MMAD(mcg)** | 0.8 | 0.9 | 0.9 |

It will be readily apparent to one skilled in the art that varying substitutions and modifications may be made to the invention disclosed herein without departing from the spirit of the invention. Thus, it should be understood that although the present invention has been specifically disclosed by the preferred embodiments and optional features, modification and variation of the concepts herein disclosed may be resorted to by those skilled in the art, and such modifications and variations are considered to be falling within the scope of the invention.

It is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items.

It must be noted that, as used in this specification and the appended claims, the singular forms "a," "an" and "the" include plural references unless the context clearly dictates otherwise.

## Claims

1. An inhaler for an inhalation formulation, comprising a canister containing a pharmaceutical composition under pressure; a metering valve for measuring a metered dose of the composition from the canister for administration to a patient in need thereof; and an actuator for actuating discharge of the metered dose to the patient; wherein the actuator includes a discharge orifice having a diameter in the range 0.2 to 0.4 mm; and wherein the pharmaceutical composition comprises an anticholinergic agent and a pharmaceutically acceptable propellant, **characterized in that** the metering valve is configured to dispense the metered dose of the pharmaceutical composition containing 2.5 to 18 micrograms of the or each anticholinergic agent.

2. An inhaler according to claim 1, wherein the anticholinergic is selected from tiotropium, ipratropium, oxitropium, aclidinium, their pharmaceutically acceptable salts or solvates, and mixtures thereof.

3. An inhaler according to claim 1 or 2, wherein the anticholinergic agent is tiotropium bromide.

4. An inhaler according to any preceding claim, wherein the propellant includes one or more pharmaceutically acceptable HFC propellants, and/or one or more pharmaceutically acceptable hydrocarbon propellants.

5. An inhaler according to any preceding claim, wherein the composition further includes a co-solvent, which is preferably a polar co-solvent.

6. An inhaler according to claim 5, wherein the co-solvent comprises one or more C₂₋₆ aliphatic alcohols and/or polyols.

7. An inhaler according to any preceding claim, wherein the pharmaceutical composition further comprises one or more beta adrenergic agents.

8. An inhaler according to any preceding claim, wherein the pharmaceutical composition further comprises one or more corticosteroids.

9. An inhaler according to claim 1, wherein the pharmaceutical composition comprises a tiotropium or a salt thereof, one or more pharmaceutically acceptable HFC propellants, and/or one or more pharmaceutically acceptable hydrocarbon propellants, and one or more pharmaceutically acceptable excipients.

10. An inhaler according to claim 9, wherein the pharmaceutical composition further comprises one or more beta adrenergic agents.

11. An inhaler according to claim 9 or 10, wherein the pharmaceutical composition further comprises one or more corticosteroids.

12. An inhaler as claimed in any preceding claim suitable for use in the treatment of a respiratory disorder.

13. An inhaler as claimed in any one of claims 1 to 11, suitable for use in the treatment of asthma and/or a chronic obstructive pulmonary disease.

## Patentansprüche

1. Inhalator für eine Inhalationsformulierung, der einen Behälter, der eine unter Druck stehende pharmazeutische Zusammensetzung enthält; ein Dosierventil zum Abmessen einer dosierten Menge der Zusammensetzung aus dem Behälter zur Verabreichung an einen Patienten, der dieser bedarf; und einen Auslöser zum Auslösen eines Ausstoßes der dosierten Menge an den Patienten umfasst; wobei der Auslöser eine Ausstoßöffnung mit einem Durchmesser im Bereich von 0,2 bis 0,4 mm aufweist und wobei die pharmazeutische Zusammensetzung ein Anticholinergikum und ein pharmazeutisch unbedenkliches Treibmittel umfasst, **dadurch gekennzeichnet, dass** das Dosierventil dazu konfiguriert ist, die dosierte Menge der pharmazeutischen Zusammensetzung, die 2,5 bis 18 Mikrogramm des oder jedes Anticholinergikums enthält, abzugeben.

2. Inhalator nach Anspruch 1, wobei das Anticholinergikum aus Tiotropium, Ipratropium, Oxitropium, Aclidinium, deren pharmazeutisch unbedenklichen Salzen oder Solvaten und Gemischen davon ausgewählt ist.

3. Inhalator nach Anspruch 1 oder 2, wobei das Anticholinergikum Tiotropiumbromid ist.

4. Inhalator nach einem vorhergehenden Anspruch, wobei das Treibmittel ein oder mehrere pharmazeutisch unbedenkliche H-FKW-Treibmittel und/oder ein oder mehrere pharmazeutisch unbedenkliche Kohlenwasserstoff-Treibmittel beinhaltet.

5. Inhalator nach einem vorhergehenden Anspruch, wobei die Zusammensetzung weiterhin ein Kosolvens beinhaltet, das vorzugsweise ein polares Kosolvens ist.

6. Inhalator nach Anspruch 5, wobei das Kosolvens ein oder mehrere aliphatische C₂₋₆-Alkohole und/oder Polyole umfasst.

7. Inhalator nach einem vorhergehenden Anspruch, wobei die pharmazeutische Zusammensetzung weiterhin eine oder mehrere beta-adrenerge Substanzen umfasst.

8. Inhalator nach einem vorhergehenden Anspruch, wobei die pharmazeutische Zusammensetzung weiterhin ein oder mehrere Kortikosteroide umfasst.

9. Inhalator nach Anspruch 1, wobei die pharmazeutische Zusammensetzung ein Tiotropium oder ein Salz davon, ein oder mehrere pharmazeutisch unbedenkliche H-FKW-Treibmittel und/oder ein oder mehrere pharmazeutisch unbedenkliche Kohlenwasserstoff-Treibmittel und ein oder mehrere pharmazeutisch unbedenkliche Hilfsstoffe umfasst.

10. Inhalator nach Anspruch 9, wobei die pharmazeutische Zusammensetzung weiterhin eine oder mehrere beta-adrenerge Substanzen umfasst.

11. Inhalator nach Anspruch 9 oder 10, wobei die pharmazeutische Zusammensetzung weiterhin ein oder mehrere Kortikosteroide umfasst.

12. Inhalator nach einem vorhergehenden Anspruch, der zur Verwendung bei der Behandlung einer Atemwegserkrankung geeignet ist.

13. Inhalator nach Anspruch 12, der zur Verwendung bei der Behandlung von Asthma und/oder einer chronischobstruktiven Lungenerkrankung geeignet ist.

## Revendications

1. Inhalateur pour une formulation d'inhalation, qui comporte un récipient contenant une composition pharmaceutique sous pression, une valve doseuse pour mesurer une dose déterminée de la composition du récipient pour une administration à un patient dont l'état le nécessite ; et un actionneur pour actionner la diffusion de la dose déterminée au patient ; dans lequel l'actionneur comprend un orifice de diffusion d'un diamètre de l'ordre de 0,2 à 0,4 mm ; et dans lequel la composition pharmaceutique comprend un agent anticholinergique et un propulseur pharmaceutiquement acceptable, **caractérisé en ce que** la valve doseuse est conçue pour diffuser la dose déterminée de la composition pharmaceutique contenant 2,5 à 18 microgrammes du ou de chaque agent anticholinergique.

2. Inhalateur selon la revendication 1, dans lequel l'agent anticholinergique est choisi parmi le tiotropium, l'ipratropium, l'oxitropium, l'aclidinium, leurs sels ou solvates pharmaceutiquement acceptables, et leurs mélanges.

3. Inhalateur selon la revendication 1 ou 2, dans lequel l'agent anticholinergique est le bromure de tiotropium.

4. Inhalateur selon l'une quelconque des revendications précédentes, dans lequel le propulseur comprend un ou plusieurs propulseurs hydrofluorocarbonés (HFC) pharmaceutiquement acceptables, et/ou un ou plusieurs propulseurs hydrocarbonés pharmaceutiquement acceptables.

5. Inhalateur selon l'une quelconque des revendications précédentes, dans lequel la composition comprend en outre un cosolvant, qui est de préférence un cosolvant polaire.

6. Inhalateur selon la revendication 5, dans lequel le cosolvant comprend un ou plusieurs alcools et/ou polyols aliphatiques en C₂-C₆.

7. Inhalateur selon l'une quelconque des revendications précédentes, dans lequel la composition pharmaceutique comprend en outre un ou plusieurs agents bêta-adrénergiques.

8. Inhalateur selon l'une quelconque des revendications précédentes, dans lequel la composition pharmaceutique comprend en outre un ou plusieurs corticostéroïdes.

9. Inhalateur selon la revendication 1, dans lequel la composition pharmaceutique comprend un tiotropium ou un de ses sels, un ou plusieurs propulseurs hydrofluorocarbonés (HFC) pharmaceutiquement acceptables, et/ou un ou plusieurs propulseurs hydrocarbonés pharmaceutiquement acceptables, et un ou plusieurs excipients pharmaceutiquement acceptables.

10. Inhalateur selon la revendication 9, dans lequel la composition pharmaceutique comprend en outre un ou plusieurs agents bêta-adrénergiques.

11. Inhalateur selon la revendication 9 ou 10, dans lequel la composition pharmaceutique comprend en outre un ou plusieurs corticostéroïdes.

12. Inhalateur selon l'une quelconque des revendications précédentes, approprié pour être utilisé dans le traitement d'un trouble respiratoire.

13. Inhalateur selon la revendication 12, approprié pour être utilisé dans le traitement de l'asthme et/ou d'une bronchopneumopathie chronique obstructive.
